# EUROPEAN PATENT APPLICATION

(11) **EP 2 060 621 A1**
(43) Date of publication of application: **20.05.2009**
(21) Application number: 06849632.2
(22) Date of filing: 27.11.2006
(51) Int. Cl.: C11B 1/00, C11B 1/06, A61K 36/899, A61P 15/00

(54) **WHEAT-GERM OIL CONCENTRATE, A METHOD FOR THE PRODUCTION THEREOF, A METHOD FOR PRODUCING WHEAT-GERM OIL AND A MEDICINAL AND PROPHYLACTIC COMPOSITION BASED THEREON AND USED FOR TREATING SEXUAL DYSFUNCTIONS**

(30) Priority: 29.08.2006 RU 2006131042
(71) Applicant: Otkrytoe Aktsionernoe Obschestvo Zavod Ekologicheskoy Tekhniki I Ekopitaniya 'Diod', Moscow 115114 (RU)
(72) Inventor: TIKHONOV, Vladimir Petrovich, Moscow, 113534 (RU)
(74) Representative: Henrion, Oliver
(86) International application number: PCT/RU2006/000630
(87) International publication number: WO 2008/026963

(57) **Abstract**

The invention relates to the field of biologically active compounds based on natural vegetable raw material, to methods for the production thereof and to the use thereof in a medical clinical and public health practice. The object of the invention is a method for producing a wheat germ oil concentrate wherein wheat germ oil is produced by cold pressing from wheat germs pre-dried to a moisture content of 6 - 8 percent, the oil is extracted twice with 92-93 vol. percent ethyl alcohol at a ratio of 1:3 and 1:2, respectively, the alcoholic extracts are combined, maintained, and after separation of the phases are evaporated under vacuum at a temperature of 50 - 60°C until the alcohol is removed. The concentrate thus obtained is used as a component for a medicinal and prophylactic agent suitable for restoring disorders in sexual functions that also contains selenium and zinc at the defined ratios. The method ensures to obtain an oil enriched with phytosterols, different forms of tocopherols and carotenoids, which increases the nutritional and healing properties thereof.

## Description

### Technical field

The invention relates to biologically active compounds based on natural vegetable raw material, to methods of production thereof, and to the use thereof in medical clinical and public health practice.

### Prior art

Thanks to its unique composition wheat germ oil is broadly used in medicine and cosmetics, but also in food industry.

The main raw material for producing wheat germ oil are wheat flakes.

According to data in the literature the following components have been found in the composition of this oil:
- Saturated fatty acids: tetradecanoic acid, palmitic acid, stearic acid, erucic acid, gondonic acid;
- Mono- and polyunsaturated fatty acids: oleinic acid, linolenic acid, linolenic acid, arachidonic acid;
- Liposoluble vitamins: tocopherols, carotenoids, ergocalciferol;
- Water-soluble vitamins: folic acid (vitamin B9), pantothenic acid.

Furthermore, lecithin, methionine, and phytosterols have been found in the composition of this oil.

Wheat germ oil is so unique because it contains at least three active complexes, comprising:
1. Antioxidants: tocopherols and carotenoids, the oil being the record holder among all natural compounds regarding its vitamin E content (200 - 600 mg percent);
2. Essential polyunsaturated fatty acids, *inter alia* linolic acid, linolenic acid, in a ratio that is optimal for the lipid metabolism in the human organism, lecithin;
3. Vitamins of the groups B, D, PP, pantothenic and folic (2 - 3 mg percent) acid, and the aminoacid methionine.

The oil has a broad spectrum of pharmaceutic and cosmetic activities (A. Nekra-sova, *"Tayny zarodyshey pshenitsy" ("The secrets of wheat germs')*, 2000).

Wheat germ oil has a universal health-improving action, it normalizes the functions of the immune system and the endocrine system. It is a stimulater of the reproductive function. It increases working ability and life tonus and strengthens the stress tolerance. It contributes to a fast healing of wounds, burns, ulcer and diseases of the gastrointestinal tract. It possesses anti-atherosclerotic and heart-protecting properties. It reduces the level of cholesterol in the blood and the liver. It regulates the hormone balance.

World's leading companies in cosmetic industry use wheat germ oil for producing creams, lotions, cosmetic masks, balms, shampoos etc.

It is known to the skilled in the art that the composition and, hence, the pharmacological spectrum and efficiency of natural products depend to a great extent on type and nature of the extracting agent, the temperature and time setting etc., and also on the ratio of the reacting substances and other aspects of the isolation process.

Therefore, there is a need for improved methods of isolation of oil from wheat germs, the oil providing a specific action, i.e., having defined nutritional, prophylactic or therapeutic properties.

A large number of methods for isolation of oil or its concentrate from wheat germs is described in prior art.

Known is a method for the production of oil and a protein product from a raw material with a low oil content, in particular from wheat germs, by pressing them in a chamber with simultaneous grinding, heating and mixing at a temperature of not more than 80°C. Prior to pressing the raw material is dried in a fluidized bed or a vibrating bed to a moisture content of 6 - 8 percent at a temperature of maximum 80°C. When during the pressing process the process temperature is reached the raw material is plasticized by addition of oil, either oil from the processed raw material, or oil that is similar in its biochemical composition (Russian patent no. 2163922).

Known is a method for the production of oil from vegetable raw material comprising extraction with hexane or other low boiling hydrocarbons that do not mix with water, separation of the miscella from the de-oiled residue by means of filtration or centrifugation and separation of the oil from the solvent by evaporation, wherein prior to said extraction the vegetable raw material is preliminarily treated once or repeatedly with an aqueous ethanol solution with a concentration that ensures a final ethanol concentration in the liquid phase obtained of 50 - 70 vol. percent, and the solid phase remaining after treatment with ethanol is extracted with hydrocarbons (Russian patent no. 2046825).

Known is Soviet patent no. 1819288 A3 which describes a method for the production of oil from wheat germs by grinding them, followed by a two-stage extraction at different temperatures, wherein as a solvent *inter alia* ethanol is used in the presence of a surfactant.

According to a further method cereal germs are ground, suspended in an aqueous medium, and heated to 30 - 40°C. An enzymatic agent with amylolytic activity is added in an amount of 1 - 5 units per 1 g of raw material, after which the temperature of the mixture is raised to 88 - 92°C. The mixture is then cooled to 40 - 50°C and treated for 1 - 2 hours at a temperature of 45 - 50°C with enzymatic agents having an amylolytic, proteolytic and cellulase activity in an amount of 0.5 - 2.0 units, 0.1 - 0.5 units, and 1.0 - 5.0 units, respectively, per 1 g of raw material. Thereafter the mixture is treated with calcium cloride (3- 5 percent of the raw material mass) at a pH value of 7.2 - 7.8, heated to 80 - 85°C, then cooled to 20°C. The solid phase sedimentates and is isolated by centrifugation. Finally the oil is extracted with ethyl alcohol at a temperature of 70 - 75°C (Russian patent no. 2092529).

All abovementioned methods provide an oil or oil concentrate with a more or less elevated concentration of vitamins and unsaturated fatty acids. However, they do not ensure the production of compositions that could be highly effectively used for prevention and treatment of sexual dysfunctions, in particular, potency disorders. It has been assumed that for this purpose an oil would be more suitable that has a high concentration of compounds of steroid structure and of antioxidants, in particular, vitamin E.

There are various methods used for isolating a fraction with compounds of steroid nature from vegetable oil. The most approachable and easiest in realization out of these methods is application of low temperature, the so-called freezing-out. However, this method has its limits, because in case if the oil has a minor content of waxes, at room temperature an opposite effect will be observed.

For isolation from vegetable oils containing lignan compounds some authors have used extraction techniques, in particular, methanol for the extraction of the phenol fraction from olive oil (Owen et al., 2000).

For production of an oil containing a concentrated sterol fraction (R. E. Ostlund et al., 2003) a new technique has been used that is based on the use of reversed-phase activated coal in the presence of ethanol.

As closest prior art to the invention can be regarded a method for the production of a wheat germ oil concentrate, comprising grinding and de-oiling the germs with ether, evaporation of the ether, and extraction of the germs with acidified water and alcohol with trichloromethane dissolved in it by means of infusion, followed by clearing, filtration and evaporation (Soviet patent no. 65874, Feb. 28, 1946).

However, said closest prior art method that, and also the other abovementioned methods are not optimal for achieving the object of the present invention, since they do not ensure a sufficiently high content of vitamin E and sterols.

### Disclosure of the invention

It is an object of the present invention to develop a new, less laborious method for the production of a wheat germ oil concentrate suitable, *inter alia,* for the production of agents for prevention and treatment of sexual dysfunctions.

Said object is achieved by a method that is **characterized in that** wheat germ oil is obtained by cold pressing wheat germs that are preliminarily dried to a moisture content of 6 - 8 percent, extracting twice with 92 - 93 vol. percent ethyl alcohol, at a ratio of 1 : 3 and 1 : 2, respectively, combining the ethanolic extracts, maintaining, and evaporating under vacuum at a temperature of 50 - 60°C after separation of the phases until removal of the alcohol.

Surprisingly ethyl alcohol of the above indicated concentration made it possible to avoid formation of an emulsion that would be difficult to separate. Furthermore, ethanol is less toxic than other organic solvents, and it is cheaper.

The oil concentrate thus obtained represents an oily viscous mass of intense yellow colour with a characteristic smell, having the following characteristics:

| | |
|---|---|
| Peroxide number | 1.2 - 1.3 mmol/kg |
| Acid number | 4.6 - 4.7 mg |
| Saponification number (KOH) | 190 - 195 mg |
| Iodine number | 110 - 112 g/100 g |
| Content of non-saponifying matter | 4.8 - 4.9 mass percent |
| Content of carotenoids | 9.45 - 10 mg percent |

The phytosterol content of the concentrate is 8 times higher, the tocopherol content is 5 times higher, and the carotenoid content is 3.7 times higher than that of the initial oil.

Below data are provided regarding the biochemical and pharmacological activity of some of the main components of the wheat germ oil concentrate:

### Vitamin E (tocopherols)

Vitamin E (tocopherols, from Greek *tocos* = birth, *pher* = to support) represents an array of 2-methyl-2(4,8',12'-trimethyltridecyl)-6-chromanol (tocol) derivatives that are biogenous substances, considerable quantities of which are present in plants and the organism of animals. Predominantly they are contained in lipoprotein cell membranes and subcellular organelles, because of the intermolecular interaction with unsaturated fatty acids.

In numerous studies conclusive evidence has been obtained for the therapeutic and prophylactic protecting activity of vitamin E (tocopherols) with regard to pathologies of the genital organs, atherosclerosis, and arteriosclerosis of the vessels.

The biological activity of α-tocopherol and its analogs is based on a high efficiency and ability to eliminate peroxidic radicals of lipids by shifting the hydrogen atom of the hydroxy group (one-electron shift) to the radical and to restore the lipids (anti-radical potential). Thus vitamin E prevents oxidation of unsaturated lipids and protects biological membranes from oxidation.

Unlike most other antioxidants vitamin E is a liposoluble (lipophil) compound, and, therefore, it can easily be incorporated directly into the structure of low density lipoproteins and cell membranes. In this way it most efficiently prevents oxidation of lipoproteins and peroxidation of lipids of the cell membranes.

However, in the reaction process with free radicals α-tocopherol oxidizes to an α-tocopheroxyl radical, and subsequently the latter transforms into the toxic α-toco-pherylquinone. This leads not only to a fast reduction of the efficient tocopherol concentration and of its antioxidant activity, but also to an increase of the pro-oxidant action which is due to the continuation of the oxidation chains lead by the tocopheroxyl radicals. It is for this reason why α-tocopherol is a weak antioxidant although it possesses a good anti-radical activity.

To enhance its anti-oxidant action the presence of other antioxidants in the medium is necessary. Therefore in most cases vitamin E is combined with the coenzyme Q and vitamin C (synergism of the antioxidant action).

In comparison to α-tocopherol, β- and δ-tocopherols possess less anti-radical activity, but their antioxidant activity is considerably higher: α-tocopherol (0.3) < β-tocopherol (0.45) < tocopherol (0.6) < δ-tocopherol (1.0).

The concentrate obtained by the present method includes the whole spectrum of forms of the vitamin, such ensuring an optimal antioxidant activity.

Table 1 shows the quantitative relationship of α-, β-, γ- and δ-tocopherols contained in the wheat germ oil concentrate obtained.

**Table 1**

| Amount of tocopherols, mg percent | Fraction of individual tocopherols in the oil, mg percent | | | |
|---|---|---|---|---|
| | α- | β- | γ- | δ- |
| 1360 | 945 | 343 | 60 | 16 |

### Phytosterols

The prevailing representatives of this class are believed to be the group of sitosterols, such as β-sitosterol, brassicasterol, capesterol, stigmasterol, ergosterol, and zymosterol. The role of this group is not completely clear. There are data showing that said sitosterols are precursors of more complex steroids.

### Poly-unsaturated fatty acids

Unlike the tissues of plants, the tissues of animals and human beings have a restricted capacity of transforming saturated fatty acids into unsaturated and poly-unsaturated fatty acids (i.e., acids having in their structure 1 - 6 double bonds).

However, unsaturated and partly poly-unsaturated fatty acids can still be synthesized in the organism by successive participation of chain extending enzymes, so-called elongases, and desaturation enzymes (i.e., that form double bonds at the respective positions of the chain), so-called desaturases, which belong to the microsomal monooxygenases (cytochrome b5).

Poly-unsaturated fatty acids are an indispensable component of the phospholipids of cell biomembranes. Poly-unsaturated fatty acids are incorporated in 2-position of the phospholipids of cell membranes and regulate their microviscosity, permeability, and electrical properties, thus creating the lipid environment of the membrane proteins and enzymes. In case of a deficiency of essential poly-unsaturated fatty acids they may be replaced in the phospholipids, also in the membrane phospholipids, by other fatty acids. In this case the properties of the structures concerned change, often in a negative way. This is especially true for somatic cells of the genital organs and spermatozoa; their membranes contain a high amount of highly unsaturated fatty acids, namely, eicosapentaenoic acid (with five double bonds) and docosahexaenoic acid (with six double bonds). The high level of unsaturation of the latter determines the specific properties of germinal cell membranes and at the same time the high level of oxidability of the phospholipids, which causes the obligatory requirement of additional incorporation, along with poly-unsaturated fatty acids, of antioxidant components (vitamins E, A, C, carotenoids, flavonoids and others) in the food of persons suffering from sexual disorders. Wheat germ oil contains practically the whole spectrum of ω-6 fatty acids: oleic acid, linolic acid, linolenic acid, and arachidonic acid, present in form of their esters, i.e., triglycerides. At the same time also a combination of a high number of antioxidants is ensured.

In order to intensify the induction mechanism of cyclic guanosine monophosphate in the smooth muscle cells of the vessels, which is necessary for improving the erectile function, along with nitrogen oxide also a sufficient production of prostaglandin PGE by the endotheliocytes is important. The prostaglandin PGE is synthesized together with other eicosanoids from arachidonic and dihomo-γ-linolenic acid by the cyclooxygenase path.

If linolic acid is provided to an organism with food or as a food supplement (e.g., in the composition of wheat germ oil), the whole spectrum of essential poly-unsaturated fatty acids and, eventually, arachidonic acid are generated from it.

Essential fatty acids are precursors of so-called eicosanic (i.e., containing 20 carbon atoms) fatty acids from which families of biologically active compounds are formed that have the most diverse effects on various aspects of metabolism. They comprise prostaglandins (PG), prostacyclins (PP), thromboxanes (TO) (summarily called prostanoids), and leukotrienes (LT).

α-Linolenic acid can be enzymatically transformed and can be a source of eicosapentaenoic acid if corresponding enzymes are sufficiently active in the organism.

To sum up, the formation of a broad spectrum of eicosanoids of various structures explains the wide range of pharmacological effects that characterize the action of poly-unsaturated fatty acids on the organism: complex influence on processes of thrombogenesis, on blood coagulability, the tone of blood vessels and bronchi, the rheological properties of the blood, the arterial tension, the immunological status, the tone of the smooth muscles of the uterus, secretion processes of the glands etc.

Obviously the broad spectrum of medicinal and prophylactic activity of the poly-unsaturated fatty acids of wheat germ oil, beyond their membrane-proctecting properties and direct initiation of the induction mechanism of cyclic guanosine monophosphate in the smooth muscle cells of the vessels, can also contribute to securing an adequate level of the sexual function in men.

### Vitamins of the groups B, D, F, pantothenic and folic acid

Preparation of samples for determination of the composition of the concentrate.

About 0.1 g (precise test portion) of the sample are placed in a serum vial and dissolved in 2 ml of a hexane/chloroform mixture (1:1), or in 2 ml chloroform. The solution thus obtained is analyzed for phytosterols and compounds of the vitamin E group.

### Determination of phytosterols by reversed phase HPLC.

A high pressure chromatograph of the company "Beckman" is used. It is equipped with an UV-detector, a Luna C18 4.6 x 150 mm column (particle size of sorbent 5 µm), and a precolumn of 20 mm length that is filled with the same sorbent. The isocratic elution mode is used (100 percent acetonitrile). The length of the detection waves is 206 nm, the flow rate of the eluent is 1.0 ml/min, the batch volume of the samples is 20 µl. For identification standard samples of ergosterol, campesterol and /3-sitosterol are used as well as recommendations (Zenkevitch et al., 2003) for identification of sterols by using logarithm values of the distribution coefficients of the studied compounds in an octanol/water system (log P), calculated on the basis of the relationship log P = f(tR). The quantitative assay is carried out by external standard method, using solutions of 0.4 mg/ml of standard samples: ergosterol (> 90 percent, Sigma) and /3-sitosterol (> 55 percent, > 40 percent campesterol, Merck). The figures regarding the phytosterol content of the oil are shown in Tables 2 and 3.

### Determination of tocopherols and tocotrienyls by reversed phase HPLC.

A high pressure chromatograph of the company "Beckman" is used. It is equipped with an UV-detector, a Luna C18 4.6 x 150 mm column (particle size of sorbent 5 µm), and a precolumn of 20 mm length that is filled with the same sorbent. lisocratic elution mode is used (isopropyl alcohol/acetonitrile 15:85). The length of the detection waves is 292 nm, the flow rate of the eluent is 1.0 ml/min, the batch volume of the sample is 20 µl. For identification standard samples of gamma-tocopherol, delta-tocopherol, alpha-tocopherol are used as well as recommendations (Zenkevitch et al., 2003) for identification of sterols by using logarithm values of the distribution coefficients of the studied compounds in an octanol/water system (log P).

The quantitative assay is carried out by external standard method, using solutions of 0.4 mg/ml of the standard samples: gamma-tocopherol (> 99 percent, Supelco), delta-tocopherol (> 99 percent, Supelco), and alpha-tocopherol (> 99 percent, Fluka).

### Analysis of an etanolic wheat germ oil extract.

A high pressure chromatograph of the company "Agilent 1100" is used that is equipped with a diode matrix detector, a Varian C18 column (4.6 x 150 mm, particle size 5 µm), and a thermostat for the column (20°C). lisocratic elution is carried out with 100 percent methanol. The sample is fed by means of an autosampler with batch volume of the sample being 20 µl.

### Determination of the total amount of carotenoids by UV-spectrophotometry.

About 0.6 g (precise test portion) of the sample are dissolved in 15 ml hexane in a measuring flask with a capacity of 25 ml, and the volume of the solution is filled up to the mark with the same solvent.

The optical density of the solution thus obtained is measured with the spectrophotometer at a wave length of 450 nm in a cuvette with a layer thickness of 10 mm. As comparison solution hexane is used.

The figures regarding the composition of the non-saponifiable fraction are shown in annexed Table 4.

The fatty acid composition of the oil is shown in annexed Table 5.

A further object of the invention is a medicinal and prophylactic composition for restoring disturbed sexual functions. Said composition can be used for normalizing the function of the testicles in men, for enhancing spermatogenesis, against impotence, and also for prevention of prostatic hypertrophy. It can further be used for stimulation of the reproductive functions in women.

As closest prior art can be regarded a biologically active additive, namely, wheat germ oil (Register of Biologically Active Supplements).

The characterizing feature of the composition consists in that it comprises a wheat germ oil concentrate obtained according to the present method, the trace element zinc, and the trace element selenium, with the following amounts of the components:

| | |
|---|---|
| Wheat germ oil concentrate | 200 - 300 mg |
| Zinc | 2 mg - 4 mg |
| Selenium in the form of Selenopyran | 15 - 20 µg (based on selenium) |

The composition is provided in standard capsules made of glycerine, ionol, gelatine, ethyl alcohol, and water.

Biochemical and pharmaceutical activity of some of the main components of the composition.
Considering the importance of vitamin E for the prevention of various pathologic processes that directly and indirectly influence the condition of male genital organs, and considering that wheat germ oil concentrate comprises a reasonable number and amount of homologs of natural tocopherol and other biologically useful substances and antioxidants, it can be assumed that the wheat germ oil concentrate represents an excellent base for a medicinal and prophylactic composition for restoring and supporting virile strength and potency. The average daily dosage of vitamin E for therapeutic and prophylactic purposes is at least 200 IU, preferably 600 IU.

As has been mentioned before, the presence of β-, γ-, and δ-tocopherol in the wheat germ oil concentrate, together with the α-tocopherol considerably increases the efficiency of the antioxidant activity of the oil. This acts especially on the membrane lipids of the cells of various tissues, including somatic cells of the genital organs and spermatozoa.

Zinc is the most important trace element the manifold action of which being due to its presence in the composition of many enzymes. Until now zinc has been detected in more than 200 metalloenzymes that are involved in most different exchange reactions, including the synthesis and decomposition of carbohydrates, fats, proteins, and nucleic acids. The zinc ion forms part of the structure of the active centre of a broad variety of enzymes, so-called metalloenzymes, a number of which stabilizes the cell membranes; it participates in the metabolism of various hormones, among others sex hormones, and in processes of the division and reactions of immune competent cells. Zinc increases the stress tolerance of organism and the resistance to catarrhal diseases as it possesses antiviral and antitoxic activity (Yu.Yu. Gitchev, *"Rukovodstvo po biologitcheski aktivnym pishtchevym dobavkam" ("Guide to biologically active food additives"),* Moscow, Triada, 2001).

I n case of a zinc deficiency the processes of spermatogenesis are suppressed, which in men expresses itself as sterility. Studies on animals showed that in 80 percent of the cases injections of zinc preparations prevent the development of cancer of the prostate gland. Zinc is also important for stabilization of the blood system, it is involved in the absorption and metabolism of one of the most important antioxidants and proliferation factor on which the growth of tissues and the condition of the skin are based, namely, vitamin A (retinol). It is known that a zinc deficiency in the liver disturbs the synthesis of the retinol binding protein (RBP) and, in result, the transport of retinol from the liver to the tissues, as zinc is comprised in the RBP which serves as the transport protein for retinol.

Many of the above indicated properties of zinc play a major role in the solution of problems of increasing the sexual function in men, as erection disorders are in many respects linked to metabolic processes in the organism and, above all, to the level of male sex hormones, the condition of antioxidant and anti-peroxide protection, and the level of the most important neurotransmitter, namely, nitrogen oxide.

The harmful effect of a zinc deficiency on the testosterone level is most pronounced in men over 35 years of age, and in advanced age it may cause prostatic hyperplasia, or even an adenoma.

The molecular basis of these processes lies in that zinc functions as an efficient inhibitor of 5a-reductase, an enzyme that controls the reduction of testosterone to dihydrotestosterone, and lowers the level of the sex hormone. An excessive formation of dihydrotestosterone, as a consequence of a zinc deficiency, leads to a reduction in erection and stimulates proliferative processes and the growth of the prostate. Furthermore, zinc blocks the hyperplastic effect of another hormone, prolactin, which plays a major role in the development of hyperplasia and prostate cancer. Zinc inhibits the action of aromatase (cytochrome P-450C19), an enzyme of the fat tissue that transforms testosterone into estradiol, and slows down binding of estrogens to the receptors, thus attenuating their negative action on the prostate.

The introduction of zinc into formulations for prevention and therapy of erectile dysfunctions caused by pathologic processes that lower the testosterone level considerably increased the efficiency of action of the composition.

The antioxidant activity of zinc is manifold. An increase of the zinc concentration in the organism lowers the concentration of peroxidic lipids, *inter alia* of low density atherogenic lipoproteins. The protective action of zinc on the kidneys is due to its antioxidant effect.

Zinc prevents the formation of especially aggressive active forms of oxygen (the OH* radical, singlet oxygen) that damage biomembranes, lipids, proteins (enzymes), and nucleic acids.

The zinc-dependent superoxide dismutase (SOD) plays a vital role in the regulation of the level of endogenous nitrogen oxide NO that is produced by the vascular endothelium cells and that is a very important vasodilating agent. As is known, free radicals that are formed in large quantities can inactivate and destroy NO. The latter being essential for an optimal functioning of the genital system as well as the cardiovascular system.

Finally, zinc is an inductor of the biosythesis of a whole class of special sulphur containing proteins, i.e., metallothioneins, that possess a very high antioxidant activity. Its antioxidant properties certainly represent an important factor as to the positive effect on the condition of skin, vision, and tissue growth, as well as for its anti-inflammatory, antiulcer and wound and ulcer healing effect. Thus, zinc belongs to antioxidants having a reparative effect.

The processes of biosynthesis of insulin, its transport, its biotransformation and effect are inseparable from the presence of a sufficient level of zinc. This is the reason for its exceptional importance for the regulating effect of insulin on the liver, and thus for metabolism as a whole, for prevention and therapy of diabetes, the latter representing a serious risk factor that may lead to erectile dysfunctions.

The zinc reserves in the organism are not large, they amount to mere 1.5 - 2 g. Zinc has been detected in all organs and tissues, but its greatest amount is found in the muscles, the liver, kidneys, prostate gland, and skin. The toxicity of zinc is not high, and even if it is administered in excess it is not accumulated, but leaves the organism. The average daily dosage of zinc is 15 mg.

In the present composition the dosage can be reduced due to a synergistic effect.

### Trace element selenium.

The importance of selenium as an essential factor of human nutrition was established about half a century ago. All over the world this problem was resolved fairly long ago in its theoretical and practical aspects, and in the repertoire of medical practitioners numerous selenium containing biologically active food additives and drugs appeared. In our country the practical clinical use of selenium is comparatively recent.

Selenium is an important trace element also for the functioning of the reproductive sphere (H. Guvenc et al., Pediatrics, 1995, v. 95(6), p. 879 - 882). The reproduction ability of the male as well as the female organism, and also the health of the child depend on an optimal consumption of selenium.

Lately the efficiency of selenium in the prevention and therapy of adenoma and cancer of the prostate in men has been demonstrated (A. M. Kamat, J. Urol., 1999, v. 161, p. 1748 - 1760).

One of the most important biochemical functions of selenium is its active part in the formation and functioning of the antioxidant system of the organism. It is comprised in the structure of major antioxidant enzymes, namely, selenium-dependant glutathione peroxidases I, II, III, and phospholipid hyperoxide glutathione peroxidase PL-GPO.

The antioxidant effect of selenium is considerably increased in combination with vitamin E, this being due to a synergism of their activity. The activity of the Se-dependtent glutathione peroxidase depends on the influence of vitamin E. The daily need of an adult for selenium is 50 - 200 µg per day, in average about 100 µg. The presence of vitamin E increases the absorbability of selenium in the duodenum and, to a smaller extent, in the jejunum and the cecum, allowing for a reduction of the dosage, thus reducing the drug stress on the organism.

Selenium helps to lower the level of lipids and glucose in the blood and of neutral fats in the liver, simultaneously mobilizing methionine. This, together with its antioxidant effect, gives rise to its anti-atherosclerotic effect. It seems to be the antioxidant and membrane stabilizing effects of selenium that lead to its advantageous influence on the condition of the liver (prevention of hepatosis and hepatitis).

Selenium influences also the metabolism of the prostatic gland by varying the prostacyclin/thromboxane ratio.

Thus, selenium represents one of the essential trace elements showing a diverse effect on metabolism and protection of the organism from the impact of various harmful factors and strengthening its resistance to unfavourable influences.

The present composition acts on the organism efficiently and softly in order to increase virile strength and to overcome erectile dysfunctions of various etiologies with the help of its components that act on several biochemical mechanisms of the erection. As basis of the composition wheat germ oil concentrate was chosen, representing a natural complex of substances that possess a multilateral biological and, above all, antioxidant effect. The concentrate comprises a large amount of various tocopherol homologs, of a number of carotenoids and essential poly-unsaturated fatty acids, plus other components. The complex of low molecular antioxidants that are capable of individually acting against singlet oxygen and peroxidic radicals of membrane lipids comprised in the oil also efficiently triggers the enzyme element of antioxidant protection. Both elements of antioxidant protection efficiently prevent oxidation of membrane lipids of the germ cells and the somatic cells of the genital organs. Besides, the enzyme element and, above all, (Zn)-superoxide dismutase, (Se)-glutathione peroxidase protect endogenous nitrogen oxide NO from transformation into toxic products, in this manner participating in the regulation of the basic release mechanism of the erection, by means of activation of guanylate cyclase and accumulation of cyclic guanosine monophosphate (cGMP), diffusing neurotransmitters into the smooth muscle cells of the cavernous tissue. The protective activity of said enzymes with respect to nitrogen oxide can be substantially reinforced by the introduction of zinc and selenium, as the latter represent cofactors of (Zn)-superoxide dismutase and (Se)-glutathione peroxidase that are capable of inactivation of especially aggressive active forms of oxygen, namely, OH* radicals, singlet oxygen, and hydrogen peroxide. Selenium's own antioxidant effect is synergistically increased by its combination with the tocopherols of the oil, the tocopherols having an advantageous influence on the activity of selenium-dependent glutathione peroxidase.

By maintaining the level of endogenous nitrogen oxide NO which is simultaneously the most important neurotransmitter in the parasympathetic nervous system, an endothelial relaxation factor of the smooth muscle layer of the cavernous bodies of the penis, and an important vasodilator, the components of the oil in the presence of zinc and selenium considerably promote blood circulation in the vessels as well as the level of erection and virile strength.

Additional incorporation of zinc in to the formulation is not only for the reason that it is a cofactor of essential antioxidant enzymes, but also for the various positive effects of this trace element on vital systems of the organism, namely, the cardiovascular system and the hormone system, both regulating the physiological mechanism of the erection. In particular, the molecular basis of the positive effect of zinc on the level of testosterone production lies in its role as an efficient inhibitor of a number of enzymes, above all, 5a-reductase. 5a-reductase controls the reduction of testosterone to dihydrotestosterone and thus lowers the level of this male hormone. An excessive production of dihydrotestosterone as a consequence of a zinc deficit stimulates decrease in erection, and stimulates proliferative processes and an enlargement of the prostate. Additionally, zinc blocks hyperplastic effects of another hormone, prolactin, that plays an important role in the development of prostatic hyperplasia and cancer. Zinc inhibits the action of the enzyme aromatase (cytochrome P-450C19) which is a specific enzyme of fat tissue that transforms testosterone into estradiol and inhibits binding of estrogens to the receptors, thus decreasing their unfavourable effect on the prostate.

Together with nitrogen oxide that triggers the mechanism of induction of cGMP in the smooth muscle cells of the vessels, for a soft supporting action on the erectile function also a sufficient production of one of the known eicosanoids, namely, the prostaglandin PGE by the endothelial cells is required. Direct precursors of the latter are linoleic, γ-linolenic and arachidonic acids, that are present in a sufficient amount in wheat germ oil.

### Short description of the drawings

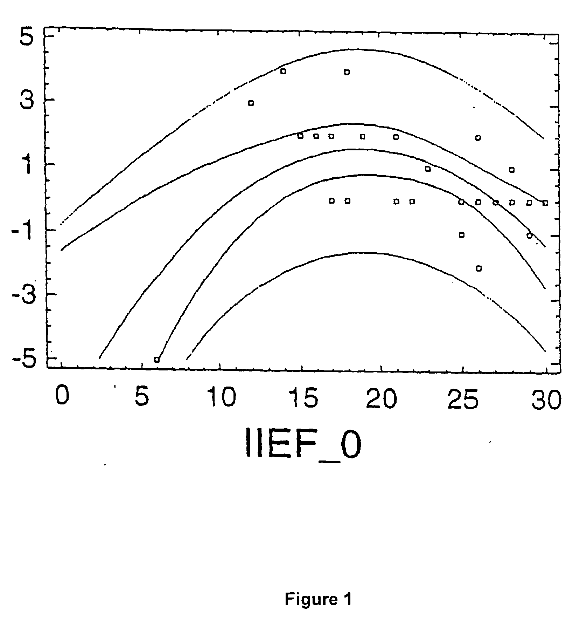

Figure 1 shows the dependency of variation of the IIEF index on its initial value.

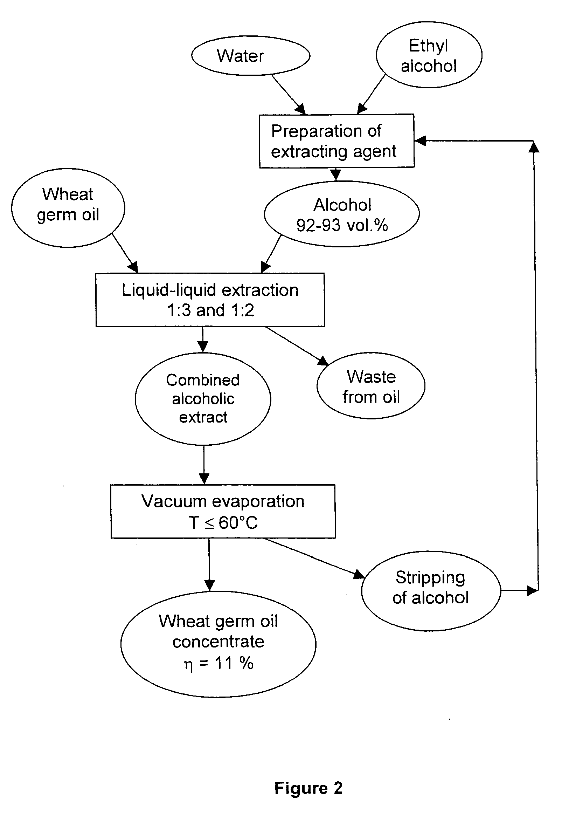

Figure 2 shows a scheme of the production process of the wheat germ oil concentrate.

### Best embodiment of the invention

### Example 1.

Wheat germs produced in a milling plant are preliminary treated, including cleaning of the germs and drying in a drier with a pseudo-bed at a temperature of 70°C for 4 - 10 min., preferably 6 minutes to a residual moisture of the germs of 5 - 9 vol. percent, preferably 8 vol. percent. The germs thus prepared are fed into a receiving bunker from where they are delivered with a conveyer and dosed into a pressing chamber where they are subjected to two-step cold pressing at a temperature of maximum 70°C, preferably 60°C, and a pressure of 120 atm in the first pressing step and 190 atm in the second pressing step. As a result of the pressing procedure a wheat germ oil is obtained that contains at least 18 vol. percent tocopherols and is appropriate for the production of a medicinal and prophylactic composition for restoring disturbed sexual functions.

### Example 2.

A clinical study was carried out by the Department of Urology of the Moscow State University of Medicine and Dentistry in the Clinical Hospital No. 50, and by the Department of Andrology and Urology of the Endocrinologic Scientific Center.

The aging process of the male organism not only brings about a general reduction of adaptability that leads to a broad range of diseases, in particular cardiovascular diseases, but is also characterized by a set of specific processes in the genital sphere which lately are used to be called "climacterium virile". In general the climacterium is a transition period in the life of a man between middle age and elderly age, this period being accompanied by a structural and functional alteration of the organism. This is a natural stage of the biological process of aging, however, it proceeds very individually. In a narrow sense the climacterium virile is, according to the definition of ISSAM, a "biochemical syndrome that occurs in mature age and is characterized by a deficiency of androgens in the blood serum which may be accompanied or not by a decrease of the sensibility of the organism to androgens, which can lead to a significant deterioration of the quality of life and can have an unfavourable influence on the functions of many systems of the organism". The experience shows that laboratory symptoms of androgen deficiency occur in 20 percent of men over 60 years of age. However, typical symptoms of late onset hypogonadism can develop also in young men as an effect of various factors that lead to chronic stress and, by virtue of a number of biochemical disorders, to androgen deficiency. The chronic fatigue syndrome or the "manager's syndrome" becomes more and more common in our time.

It must be noted that the evaluation of the level of androgen saturation of a male organism represents a complex task. This is due on the one hand to significant variations of the androgen level in the male organism in the course of a day (up to 30 percent) that render determination of the real testosterone content in the blood plasma difficult, and on the other hand to the absence of a direct relationship between the level of testosterone in the blood plasma and clinical symptoms. The explanation for the latter are changes in the sensitivity of the receptors of the target organs to testosterone in aged men. Often clinical manifestations of androgen deficiency can be observed even if the testosterone level is normal. Typical is also a moderate reduction of the testosterone concentration in the blood plasma with normal values of the gonadotropic hormones. The results of recent scientific studies provide evidence for the important role of testosterone in sustaining the normal elasticity of the collagen fibers of the cavernous tissue and the synthesis of NO, the latter being a central mediator of the "erection". It as been demonstrated that even relatively short periods of decrease of testosterone under a threshold level (plasma T: 10 - 13 nmol/l, free T: 200 - 250 pmol/l) can irreversibly influence the condition of the cavernous tissue and cause its sclerosis.

The efforts of modern medicine in the field of correction of said disorders are currently focused on the development of an androgen-substitution therapy (AST) and of new testosterone preparations. Today the generally available drug formulations for AST are in form of buccal or oral preparations, preparations for intramuscular application (prolonged and short action), in form of implantable slow-release pills with long-term effect, skin plasters and gels.

However, despite the variety of drug formulations there is still a number of unresolved problems. First, none of the available preparations is capable of precise reproduction of the circadian rythm of the testosterone level in the blood plasma. Second, just like most medicinal preparations, androgens may develop undesired side effects. This concerns in particular their effect on the liver, prostate gland, lipid profile, cardiovascular system, hematological status, sleep system, social behaviour, and emotional condition. Special attention must be paid to an early diagnostics of prostate cancer in the context of AST.

The above mentioned problems directed the attention on the study of drug preparations that contribute to a sustained synthesis of endogenous testosterone on a normal level and to support its biological effects and thus prevent development of secondary hypogonadism. Among others, special attention was directed on anti-oxidant preparations, in particular vitamins A and E, and some trace elements (selenium). An advantageous feature of these substances consists in that they are contained in many food products, making it possible to control their intake in the context of a diet, without the need of administration of chemically synthesized preparations. Due to the above mentioned properties a long-term prophylaxis of metabolic disorders is possible.

Objects of the study:
1. Evaluation of the effect of the present composition on the copulative function in men (erectile function and spermatogenesis).
2. Evaluation also of its effect on the androgenous state of men and determination of possible mechanisms that cause its alteration.

Criteria for participation in the test:
1. Men aged from 30 to 65 years.
2. Total testosterone level under 12 nmol/l.
3. Clinical symptoms of androgen deficiency, ASM (androgenous state of man) confirmed by enquirer.
4. Heterosexual.
5. Existence of permanent sexual partner.
6. Possibility execute the protocol of the study.

Criteria for exclusion from the test:
1. Endocrine disorders that lead to a reduced testosterone secretion, namely, hypothyroidism, thyrotoxicosis, hyperprolactinemia (carried out on the basis of determination of the levels of TTH, free T4, free T3, prolactin), diabetes mellitus.
2. Intake of preparations that influence the androgenous state: androgens, chorionic gonadotropin; 5α-reductase inhibitors, dihydroepiandrosterone.
3. Use of other preparations that stimulate the erectile function (e.g., PDE-5 inhibitors, intracavernous preparations, phytopreparations etc.) or participation in a therapy (including reflexotherapy, psychotherapy etc.) at the time of the selection and/or during the presumed period of the study.
4. Intake of preparations that are known for their inhibitory effect on the erectile function (psychoactive drugs, β-adreno-blockers, anti-androgens, NSAIDs, second-generation histamine H-1 receptor blockers etc.).
5. Organic diseases that reduce the erection (Peyronie's disease, coronary artery disease, chronic alcohol intoxication etc.).
6. Organic diseases that inhibit spermatogenesis (varicocele, cryptorchism, obstruction of the spermaducts etc.).
7. Known allergy to any of the components of the preparation.
8. Acute diseases at the moment of the selection.
9. Other, chronic, diseases that according to the medical doctor's opinion might influence on the result of the study (including infectious diseases, organ failure, a recent acute cerebrovascular accident or craniocerebral trauma, neurological diseases with state of decompensation etc.).
10. Infection with HIV, hepatitis B, or hepatitis C virus.
11. Sexually transmitted infections.
12. Congenital or acquired anatomic defects in the genital system preventing a normal sexual function.

Design of the study.
Open study, without control by the use of-placebos, on 30 men suffering from late onset (age-related) hypogonadism.

Preliminary examination over 2 weeks (check of the criteria for participation or exclusion, declaration of consent to participation in the study, history taking, diagnosis of the general condition of organs and systems, general clinical and laboratory tests).

Therapeutic phase over 4 weeks, with visits every 2 weeks (± 2 days) for evaluation of the IIEF-5 (EF) and DAN-PSS-Sex values, hormone tests, checking the tolerance to the composition, identification of side effects and changes of the general condition of the patient. The final evaluation of the hormone status takes place after 30 ± 3 days, and the final visit is after 80 - 90 days when the values of spermatogenesis are determined.

Instruments used:
1. International Index of Erectile Function (version for EF) IIEF-5
2. AMS questionnaire on symptoms of aging in men
3. DAN-PSS-Sex scale

### Evaluation of the results.

Variables analyzed:
1. Changes of the values of the AMS scale
2. Changes of the IIEF (EF) values
3. Changes of the spermogram values
4. Development of the results of the hormone tests

The statistical analysis of the data obtained was carried out by various methods, using the software package Statgraphics 5.0 Plus.

### RESULTS OF THE STUDY

According to the criteria for participation in the study 30 men were selected showing symptoms of androgen deficiency. The average age of the patients was 37.5 years (Fig. 4).

These patients were observed at hospital and outpatiently. The main reason for consultation of an urologist were not always sexual disorders. In some cases the erectile dysfunction was recognized during the examination in connection with other disorders.

According to the design of the study all patients gave their consent to participate in the study, the history was taken, the general condition of organs and systems was checked, and general clinical and laboratory tests were carried out. Furthermore, the basic condition of the copulative function was assessed by use of the scale of the International Index of Erectile Function and the DAN-PSS-Sex scale, hormone tests were conducted, and the ejaculate was examined. The values of these characteristics that were obtained during the intermediate visits were compared to the initial values. According to the protocol of the study men were selected for the study who had shown a reduced level of total testosterone, which, measured in the morning, was lower than 12 ng/ml. The results of the analysis of the data obtained by use of the AMS scale showed that 12 individuals had weakly expressed androgen deficiency symptoms, while in the other 18 individuals the symptoms were moderate. As concomitant organical diseases were a criterion for exclusion from the study we are of the opinion that the androgen deficiency (considering the fairly juvenile age of the patients) in all patients bore stress character (chronic fatigue syndrome, manager's syndrome etc.). In the anamnesis of all patients considerable physical and mental stress has been observed.

The main symptoms about which the patients complained, were: deterioration of health and general condition, increased sweating, sleeplessness, irritability, muscle weakness. Some patients mentioned a reduction of sexual activity and of the quality of the erection, a shorter duration of the coitus, a weakening of the orgasmic sensation.

By analysis of the patients's answers to the IIEF questionnaire it was found that 1 (33.3 percent) individual suffered from an expressed disorder of the erectile function, 4 (13.3 percent) individuals mentioned a moderately expressed disorder of the erectile function, 8 (26.6 percent) a weak to moderate disorder, 7 (23.3 percent) a weak disorder, and 10 (33.3 percent) of the individuals showed values within the normal limits (Fig. 5).

According to the criteria for participation in the study patients who had clear organic reasons for erectile dysfunctions were not included. However, in order to entirely exclude other possible factors, apart from androgen deficiency, special examination methods were applied, in particular triplex ultrasound Doppler sonography of the penile vessels with a pharmacological test, assessment of nocturnal penis tumescence (Rigiscan), and cavernosography. On the basis of the values obtained the general neurological condition of the patient was examined.

During the examinations in none of the cases clinically significant organic changes as to the penis were found. This consented the assumption that the erectile dysuncion (if occurred), and also androgen deficiency, were caused by chronic stress.

We also paid attention to concomitant diseases that must be taken into account during examination and treatment of the patient. The results are shown in the Table (Table 6).

**Table 6**

| Concomitant diseases | |
|---|---|
| Nosology | Number of Patients |
| Chronic prostatitis | 1 |
| | 1 |
| Gleet | 5 |
| Urolithiasis | 6 |
| COPD | 3 |
| Gallstone disease | 2 |
| Gastric and duodenal ulcer | 3 |
| Chronic colitis | 3 |
| Chronic pancreatitis | 4 |
| Skin diseases (psoriasis) | 1 |
| Varicose veins in the lower limbs | 5 |

Prior to starting the intake of the preparation the ejaculate of all patients was analyzed. The results showed spermatogenic disorders of various degrees in 15 cases (50 percent), characterized by a decreased spermatozoa motility, a decreased ejaculate volume and a higher viscosity and longer liquefaction period (we call this "viscous semen syndrome"), while 10 patients were observed and treated for idiopathic infertility.

After the first examination and check-up of the patients as to compliance with the criteria for participation in the study the patients were given the test composition (wheat germ oil 250 mg, selenopyran 15 µg (based on selenium), zinc 5 mg), and the treatment began. We did not prevent the patients from taking other preparations if required for treatment of concomitant diseases (except for drugs included in the list of criteria for exclusion from the study).

According the protocol each patient took orally 2 capsules 3 times a day over a period of 4 weeks. During each visit of the patients an intermediate evaluation of the erectile function and the general health condition was carried out with the use of the above mentioned instruments, and possible side effects of the drug were identified. After 3 months the ejaculate was analyzed again.

The analysis of the results of the AMS scale after conclusion of the course of treatment with the drug (i.e., after 4 weeks) showed a reliable reduction of the symptoms of androgen deficiency (p = 0.0004) (Fig. 6).

As can be seen from the data, the average reduction of the total rating according to the AMS scale was 5.9 (15.6 percent).

In the following the results of the hormone tests are presented that were carried out prior to the study and immediately after termination of the drug intake course (Table 7).

**Table 7**

| Table of the hormone indices prior to treatment and after treatment | | | | |
|---|---|---|---|---|
| Parameter | Prior to treatment | After 30 days of study | Normal parameters | P |
| TTH | 0.9 ± 0.4 | 1.1 ± 0.6 | 0.25 - 3.5 mU/l | 0.34 |
| Free T4 | 15.6 + 1.2 | 16.7 ± 2.2 | 9.0 - 20.0 pmol/l | 0.47 |
| Prolactin* | 640 ± 105 | 480 ± 60 | 60 - 510 mU/l | 0.045 |
| Testosterone* | 8.2 ± 2.5 | 15.9 + 3.7 | 11.0 - 33.5 nmol/l | 0.021 |
| Estradiol | 81 ± 40 | 90 + 50 | 20 - 240 pmol/l | 0.07 |
| SSBG* | 56.7 + 10.3 | 34.3 ± 6.8 | 12.9 - 61.7 pmol/l | 0.035 |
| LH | 2.1 +0.5 | 3.4+0.8 | 2.5 - 11.0 U/l | 0.32 |
| FSH | 6.7 + 2.2 | 7.2 + 1.8 | 1.55 - 9.74 U/l | 0.55 |
| IRI | 28.4 ± 2.1 | 25.2 + 1.6 | 2.3 - 26.4 µU/ml | 0.06 |
| Leptin | 35.2 ± 10.2 | 22.3 ± 12.2 | up to 12 ng/ml | 0.042 |

| | | | | |
|---|---|---|---|---|
| * variations statistically valid | | | | |

The results of the study show that in association with the intake of the composition in all men a significant increase of the testosterone concentration was noted, against the background of a lower prolactin and leptin level.

The following data demonstrate the changes of the parameters of the IIEF scale during the intake of the present composition and at the end of the treatment course (Fig. 7). As can be seen from the diagrams presented below, we did not note any significant change of the total average rating.

However, by using the polynomial regression method we succeeded in finding a common pattern in the changes of the average IIEF rating which is presented below (Fig. 8).

The diagrams reveal that the influence of the treatment on the erectile function in men was dependant on its initial state: the more expressed the disorders of the erectile function were that the patient had prior to treatment, the more often a positive result was observed. Thus, in the group of patients with moderate erection disorders an improvement of the parameters of the IIEF index (in average 19.3 percent) was noted in all of the four patients (100 percent). In the group of men whose erectile dysfunction was on the threshold between weak and moderate symptoms, the increase of the average rating according to the IIEF scale (in average 13.3 percent) was observed only in four individuals (50 percent). Among the patients with weak erection disorders an improvement by 4.3 percent of the parameter was noted in 2 individuals (28.6 percent). And finally, among the men who technically did not suffer from erection disorders, two patients (20 percent) also showed an increase of the average IIEF rating, the medium increase being 5.6 percent.

Said changes of the hormone status, and also the results obtained by way of the questionnaires, corresponded to the subjective impression of the patients who reported:
- disappearance or significant decrease of adynamia,
- normalization of sexual desire,
- improvement of erection,
- increase of working ability and physical activity,
- improvement of general health condition,
- mood improvement,
- reduction of quantity of fat tissue, manifest from a reduction of waist circumference in average by 6.1 cm in one month.

The following data reflect the changes found by analysis of the ejaculate (Table 8).

**Table 8**

| Paramenters of ejaculate prior to treatment and after treatment (evaluation 3 months after treatment) | | | | |
|---|---|---|---|---|
| Parameters | Prior to treatment | After treatment | Normal parameters | P |
| Ejaculate volume* | 1.4 + 0.5 | 2.8 ± 0.8 | 2.0 - 6.0 ml | 0.003 |
| Liquefaction* | > 30 million | < 30 million | 10 - 30 million | - |
| Viscosity* | 1.0 ± 0.3 cm | 0.3 ± 0.2 cm | 0.1 - 0.5 cm | 0.002 |
| Number of spermatozoa per 1 ml | 30 + 11 | 32 ± 14 | > 20 million | 0.45 |
| Motility a + b* | 10 + 10 | 40 ± 20 | > 50 percent | 0.04 |
| Number of leukocytes | 2.3 + 1.1 | 2.1 + 1.2 | < 1 million | 0.34 |

The data of the study demonstrate a significant increase of spermatozoa motility without any effect on the parameter of leukocytes in the ejaculate. Furthermore, an increased ejaculate volume and a shorter liquefaction period were noted. All specified parameters are dependent on androgen, suggesting an androgen-mediated influence on spermatogenesis.

During the intake no single case of side effect of the preparation was observed.

### Industrial applicability

1. The preparation is suitable for efficient treatment of men suffering from acquired stress-induced androgen deficiency.
2. The increase of the testosterone concentration in connection with a reduction of the prolactin and leptin levels supposes as central (hypothalo-hypophyseal) feature of the composition's action, but also as peripheral action, a reduction of the fat tissue. The latter leads to less aromatization of testosterone to androgens. The reduction of the level of globulin that binds sex steroids has an additional positive effect and increases the level of free testosterone.
3. In men suffering from acquired stress-induced androgen deficiency sexual desire is restored, and the sexual function is significantly improved.
4. The parameters of spermatogenesis in men suffering from idiopathic infertility are improved (among the cases of infertility more than 50 percent of the cases represent idiopathic infertility), leading to a higher spermatozoa motility and a shorter ejaculate liquefaction period. The positive effect on the spermatogenesis parameters is likely due to the increased testosterone concentration which is the main hormone influencing spermatozoa maturation and ejaculate liquefaction.
5. The composition can be recommended as a drug for a single agent therapy as well as for a combined therapy for treatment of idiopathic infertility and psychogenic disorders of the sexual function.

The data obtained in the clinical study permit the preparation to be considered an efficient and safe drug, capable of relieving the symptoms of stress-induced androgen deficiency, including disorders of the copulative function. Thus, the composition may be recommended for use in said category of patients.

**Table 4. Summary of data obtained by chromatography-mass-spectrometric analysis of the composition of non-saponifiable fractions**

| Sample (content of non-saponifiable fraction, percent) | Total content of compounds of various chemical groups, mass percent of non-saponifiable fraction | | | |
|---|---|---|---|---|
| | Carboxylic acids | Squalene | Isoperiodic compounds of all classes (phytol / **isophytol**) | Carbonyl compounds |
| Wheat germ oil (2.7) | 27.4 | 1.3 | 43.0 (31.9) | 13.1 |
| Wheat sprout oil (Austria) (2.0) | 12.1 | 1.0 | 24.8 (44.2) | 4.2 |
| Wheat germ oil "Osoboe" - vitamin E (3.9) | 43.8 | 0.8 | 40.9 (25.2) | 3.6 |
| Bran oil (2.0) | 30.8 | - | ∼ 44 (« 32) | - |
| Preparation | | | | |
| "Prostamo)-Uno"(1.9) | 23.7 | 19.2 | 31.7(11.5) | 13.6 |
| Palmetto extract (2.1) | 28.1 | 26.3 | 31.0 | 12.5 |
| Raisin-seed oil (0.6) | 54.0 | - | - | - |

| | | | | |
|---|---|---|---|---|
| *Notice:* The dash in the table means that the corresponding component was not identified in the sample. | | | | |

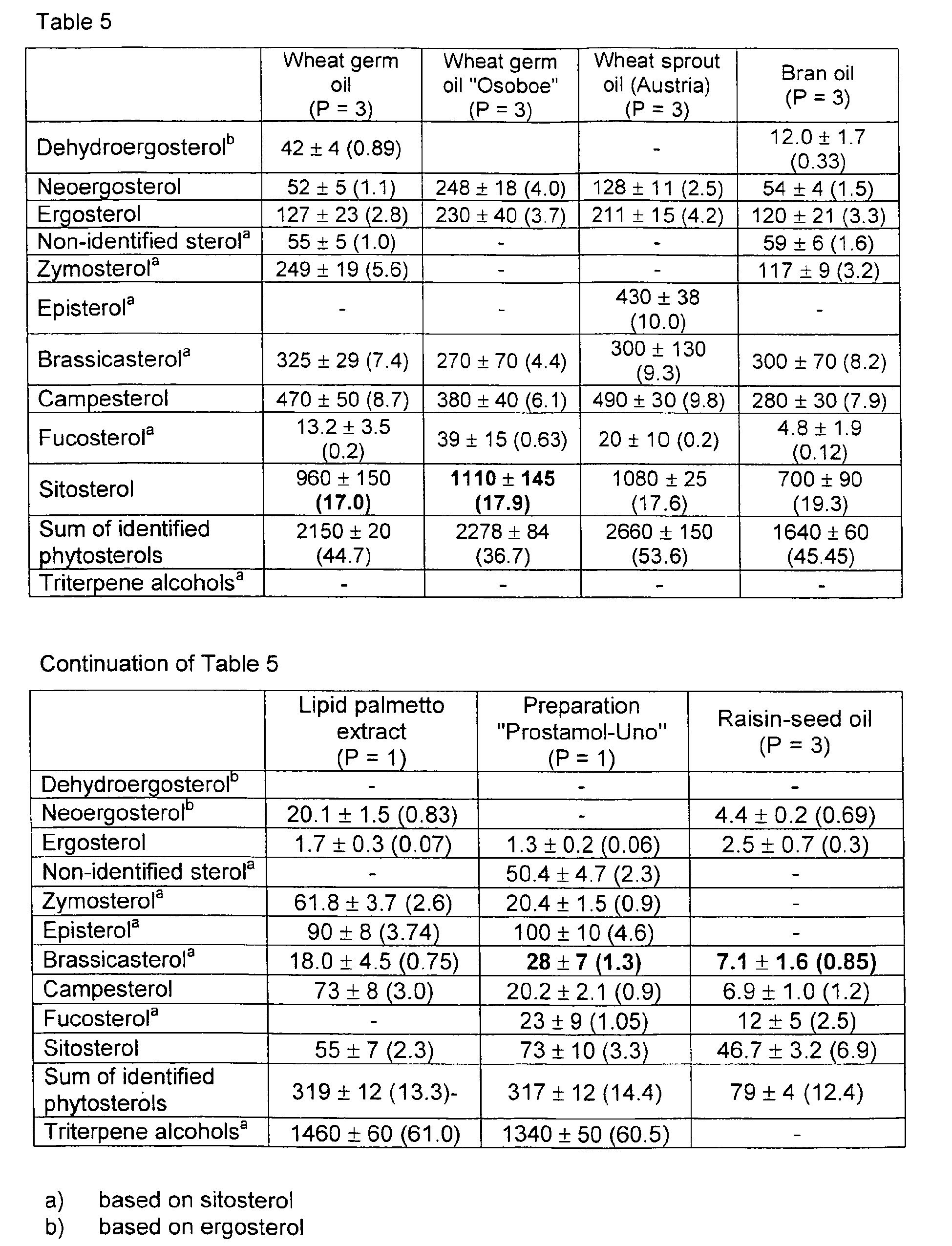

## Claims

1. Method for the production of wheat germ oil with a content of tocopherols of at least 18 vol. percent, comprising cleaning of the germs, drying, pressing and filtration, wherein cleaning and drying are performed in a dryer with a fluidized bed for a period of 4 - 10 min., followed by a two-step cold pressing of the germs at a temperature not exceeding 70°C and a pressure of not less than 120 atm and not exceeding 200 atm in the first and second step, respectively.

2. Method for the production of a wheat germ oil concentrate, **characterized in that** an oil is produced by means of cold pressing from wheat germs pre-dried to a moisture content of 5 - 9 percent, the oil is extracted twice with 92-93 vol. percent ethyl alcohol at a ratio of 1:3 and 1:2, respectively, the alcoholic extracts are combined, maintained, and after separation of the phases are evaporated under vacuum at a temperature of 50 - 60°C until removal of the alcohol.

3. Wheat germ oil concentrate, **characterized in that** it is produced by the method according to claim 2 and possesses the following characteristics:
| | |
|---|---|
| Peroxide number | 1.2 - 1.3 mmol/kg |
| Acid number | 4.6 - 4.7 mg |
| Saponification number (KOH) | 190 - 195 mg |
| Iodine number | 110 - 112 g/100 g |
| Content of non-saponifying matter | 4.8 - 4.9 mass percent |
| Content of carotenoids | 9.45 - 10 mg percent. |

4. Medicinal and prophylactic composition for restoring disorders in sexual functions based on wheat germ oil, **characterized in that** it comprises a wheat germ oil or wheat germ oil concentrate according to claims 1 and 3 and additionally a complex of trace elements, comprising zinc and selenium in form of selenopyran, with the following amounts of the components (mg):
| | |
|---|---|
| Wheat germ oil concentrate | 200 - 300 mg |
| Zinc | 2 mg - 4 mg |
| Selenium in form of selenopyran | 0.015 - 0.020 mg (based on selenium) |

5. Composition according to claim 4, **characterized in that** it is in the form of capsules.
